# EUROPÄISCHE PATENTSCHRIFT

(11) **EP 0 962 450 B1**
(45) Veröffentlichungstag und Bekanntmachung des Hinweises auf die Patenterteilung: **08.01.2003**
(21) Anmeldenummer: 99109892.2
(22) Anmeldetag: 20.05.1999
(51) Int. Cl.: C07C 319/14, C07C 323/09, C07C 323/36, C07C 323/20

(54) **Verfahren zur Herstellung von Perfluoralkyl-aryl-sulfiden**
Process for the preparation of perfluoalkylaryl sulfides
Procédé pour la préparation de disulfures de perfluoroalkylaryl

(30) Priorität: 02.06.1998 DE 19824488
(43) Veröffentlichungstag der Anmeldung: 08.12.1999
(73) Patentinhaber: Bayer Aktiengesellschaft, 51368 Leverkusen (DE)
(72) Erfinder: Popkova, Vera-Yakovlevna, Prof.Dr., 117421 Moscow (RU); Marhold, Albrecht, Dr., 51373 Leverkusen (DE)

(56) Entgegenhaltungen:
- EP-A- 0 733 614
- B.QUICLET-SIRE ET AL: TETRAHEDRON LETT., Bd. 37, Nr. 50, 1996, Seiten 9057-9058, XP004070557

## Beschreibung

Die vorliegende Erfindung betrifft ein verbessertes Verfahren zur Herstellung von Perfluoralkyl-aryl-sulfiden.

Fluorhaltige Substituenten tragende aromatische Verbindungen sind Zwischenprodukte für die Synthese von Agrochemikalien, Pharmazeutika, Farbstoffen und Polymeren. Insbesondere die Trifluormethylthio-Gruppe wird in die Entwicklung neuer biologisch aktiver Verbindungen und neuer Farbstoffe einbezogen, weil sie, bei hoher chemischer Stabilität, der lipophilste bekannte Substituent ist (R.E. Banks, K.C. Lowe: "Fluorine in Medicine in the 21^{st} Century", Chemserve Ltd., Manchester (1994), S.B. Walker: "Fluorine Compounds as Agrochemicals", Fluorochem Ltd., Old Glossop (U.K.) (1990), EP-A 374 061, J.H. Clark, D. Wails, T.W. Bastock: "Aromatic Fluorination", CRC Press, Boca Raton, F1 (1996), Seite 119). Beispielsweise wird 2-Hydroxyphenyl-trifluormethyl-sulfid zur Herstellung von bestimmten Herbiziden benötigt (US-A 4.581.059). Chlor-, nitro- und aminosubstituierte Phenyltrifluormethylsulfide werden als Zwischenprodukte für Pestizide eingesetzt (DE-A 37 37 984 und DE-A 37 37 985).

Es sind bisher mehrere Methoden zur Herstellung von Perfluoralkyl-aryl-sulfiden bekannt geworden. Die meisten davon benötigen teure und/oder toxische Reagenzien, was für eine technische Produktion nachteilig ist Man kann Pentafluorethyl und längerkettige Perfluoralkylradikale in aromatische Thiole durch ionenradikalische und kationische Reaktionen mit Perfluoralkyliodiden einführen (J. General Chem. USSR, 53, 2254 (1983) und J. Org. Chem. USSR, 13, 1985 (1977)). Perfluoralkyliodide haben jedoch den Nachteil, daß sie in aufwendiger Weise hergestellt werden müssen und deshalb teuer sind.

Für die C₁-Derivate gibt es eine neuere Methode (Tetrahedron Lett. 37, 9057 (1996)), gemäß der eine thermisch induzierte Decarboxylierung von Kaliumtrifluoracetat in der Gegenwart von Arylsulfiden durchgeführt wird. Obwohl dieses Verfahren einen deutlichen Fortschritt zu den älteren Methoden darstellt, sind die Ausbeuten mit größtenteils nur um 50 % d.Th. noch unbefriedigend, und es ist bisher auf die Einführung der Trifluormethylgruppe beschränkt. Weiterhin kann dieses Verfahren in technischem Maßstab praktisch nicht durchgeführt werden, weil dann die stark exotherme Reaktion praktisch nicht mehr zu kontrollieren ist. Es wird nämlich beschrieben, daß man das gesamte Reaktionsgemisch durch in Kontaktbringen mit einem heißen Wärmeträgermedium rasch erwärmt. Dabei setzt dann eine stark exotherme Reaktion ein. Über den Druck werden keine Angaben gemacht. Der Fachmann geht deshalb davon aus, daß bei Normaldruck gearbeitet wurde.

Es wurde nun ein Verfahren zur Herstellung von Perfluoralkyl-aryl-sulfiden der Formel (T) gefunden

Ar-S-R^{f} (I),

in der
- Ar: für gegebenenfalls substituiertes Phenyl, Naphthyl, Pyridyl oder Pyrimidyl steht, wobei die Molekularmasse dieses Radikals, gegebenenfalls vorhandene Fluoratome nicht mitgerechnet, kleiner als 200 g/mol ist, und
- R^{f}: für ein Perfluoralkylradikal mit 1 bis 7 C-Atomen steht,
durch Umsetzung von Disulfiden der Formel (II)

Ar-S-S-Ar (II),

in der Ar die bei Formel (I) angegebene Bedeutung hat,
mit Alkalisalzen aliphatischer Perfluorcarbonsäuren der Formel (III)

R^{f}-COO⁽⁻⁾M⁽⁺⁾ (III),

in der
- R^{f}: die bei Formel (I) angegebene Bedeutung hat und
- M: für ein Alkalimetall steht,
in Gegenwart eines aprotischen Lösungsmittels umsetzt, das dadurch gekennzeichnet ist, dass man
- als aprotisches Lösungsmittel solche einsetzt, die einen höheren Siedepunkt als das jeweilige Produkt der Formel (I) haben und
- bei einem Druck von 1 bis 500 mbar und einer Temperatur von 120 bis 250°C arbeitet und
- das gebildete Produkt der Formel (I) in dem Maße abdestilliert, wie es sich bildet.
   - Ar: steht bevorzugt für gegebenenfalls einfach oder mehrfach, unabhängig voneinander durch Halogen, Nitro, Cyano, gegebenenfalls durch Fluor und/oder Chlor substituiertes C₁-C₆-Alkyl oder gegebenenfalls durch Fluor und/oder Chlor substituiertes C₁-C₆-Alkoxy substituiertes Phenyl, Naphthyl, Pyridyl oder Pyrimidyl.
   - R^{f}: steht bevorzugt für ein Perfluoralkylradikal mit 1 bis 5 C-Atomen.
   - Ar: steht besonders bevorzugt für gegebenenfalls einfach oder mehrfach unabhängig voneinander durch Fluor, Chlor, Brom, Nitro, Cyano, gegebenenfalls durch Fluor und/oder Chlor substituiertes C₁-C₄-Alkyl oder gegebenenfalls durch Fluor und/oder Chlor substituiertes C₁-C₄-Alkoxy substituiertes Phenyl, Naphthyl oder Pyridyl.
   - R^{f}: steht besonders bevorzugt für ein Perfluoralkylradikal mit 1 bis 4 C-Atomen.
   - Ar: steht ganz besonders bevorzugt fiir gegebenenfalls ein- oder zweifach, unabhängig voneinander durch Fluor, Chlor, Brom, Nitro, Cyano, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Trifluormethoxy, Difluormethoxy, Chlordifluormethoxy, 2-Chlor-1,1,2-trifluorethoxy, 1,1,2-Trifluorethoxy, 2,2,2-Trifluorethoxy, 1,1,2,2-Tetrafluorethoxy, 2,2,2-Trichlor-1,1-difluorethoxy, 1,1-Difluorethoxy, Trifluormethylthio, Difluormethyl, Chlordifluormethyl, 2-Chlor-1,1,2-trifluorethyl, 1,1,2-Trifluorethyl, 1,1,2,2-Tetrafluorethyl oder Pentafluorethyl substituiertes Phenyl, Naphthyl, 2-Pyridyl, 3-Pyridyl oder 4-Pyridyl.
   - R^{f}: steht ganz besonders bevorzugt für Trifluormethyl, Perfluorethyl oder Perfluorpropyl.

Die vorliegende Erfindung betrifft weiterhin ein Verfahren zur Herstellung von Perfluoralkyl-aryl-sulfiden der Formel (I')

Ar'-S-R^{f} (I'),

in der
- Ar': für durch Hydroxy, Mercapto oder Amino substituiertes Phenyl, Naphthyl, Pyridyl oder Pyrimidyl steht, wobei die Molekularmasse dieses Radikals kleiner als 200 g/mol ist und
- R^{f}: die bei Formel (I) angegebene Bedeutung hat,
durch Umsetzung mit Disulfiden der Formel (II')

Ar"-S-S-Ar" (II'),

in der
- Ar": für durch Methoxy, Methylthio oder Nitro substituiertes Phenyl, Naphthyl, Pyridyl oder Pyrimidyl steht, wobei die Molekularmasse dieses Radikals kleiner als 200 g/mol ist,
mit Alkalisalzen aliphatischer Perfluorcarbonsäuren der Formel (III) in Gegenwart von aprotischem Lösungsmittel, das dadurch gekennzeichnet ist, dass man
- als aprotisches Lösungsmittel solche einsetzt, die einen höheren Siedepunkt als das jeweilige Produkt der Formel (I) haben,
- bei einem Druck von 1 bis 500 mbar und einer Temperatur im Bereich von 120 bis 250°C arbeitet und
- das gebildete Produkt der Formel (I")

   Ar"-S-R^{f} (Iʺ),

   in der
   - Ar": die bei Formel (II') und
   - R^{f}: die bei Formel (I) angegebene Bedeutung haben,
in dem Maße abdestilliert, wie es sich bildet und anschließend durch eine an sich bekannte Spaltung der Methoxy- oder Methylthiogruppe bzw. durch eine an sich bekannte Reduktion der Nitrogruppe Perfluoralkyl-aryl-sulfide der Formel (I') erhält.

Vorzugsweise ist die Molekularmasse der Arylradikale Ar, Ar' und Ar", gegebenenfalls vorhandene Fluoratome nicht mitgerechnet, kleiner als 180 g/mol, besonders bevorzugt kleiner als 160 g/mol.

Die zur Durchführung der erfindungsgemäßen Verfahren benötigten Arylsulfide der Formeln (II) und (II') sind zum Teil kommerziell erhältlich oder lassen sich nach oder analog zu bekannten Verfahren herstellen (s. Synth. Commun. 25, 227 (1995) und 26, 191(1996), Chem. Lett. 12, 2269 (1990), Khim. Geterots. Soed. N9, 1276 (1992) [Engl. Transl.: Chem Heteroc. Comp. 28, 1084 (1992) und Zh. Org. Khim. 20, 202 (1984) [Engl. Transl.: J. Org. Chem USSR, 20, 181 (1984)]).

Die weiterhin als Ausgangsmaterialien benötigten Alkalisalze von Perfluorcarbonsäuren der Formel (III) sind ebenfalls zum Teil kommerziell erhältlich oder lassen sich auf im Prinzip bekannte Weise durch Neutralisation aus den entsprechenden Perfluorcarbonsäuren herstellen. Bevorzugt setzt man die Natrium- oder Kaliumsalze (M = Na oder K), besonders bevorzugt die Kaliumsalze ein.

Im allgemeinen setzt man pro mol Disulfid der Formeln (II) oder (II') 1,0 bis 5 mol, vorzugsweise 1,5 bis 2,5 mol Alkalisalz einer Perfluorcarbonsäure der Formel (III) ein.

Die erfindungsgemäßen Verfahren werden in Gegenwart von aprotischem Lösungsmittel durchgeführt. Hierfür kommen z.B. Lösungsmittel mit einem Siedepunkt bei Normaldruck von über 210°C, vorzugsweise über 250°C in Frage. Nach oben ist deren Siedepunkt prinzipiell nicht begrenzt. Das Lösungsmittel sollte nur eine für die Rührbarkeit der Reaktionsmischung ausreichende niedrige Viskosität bei Reaktionstemperatur besitzen. Die Bezeichnung Lösungsmittel beschränkt die Anwendbarkeit nicht auf solche mit vollständiger Lösefähigkeit fiir die Edukte bei der jeweils eingesetzten Menge. Die Lösungsmittel müssen die Edukte jedoch wenigstens teilweise zu lösen vermögen. Beispielsweise kann man Tetramethylensulfon, Triethylenglykoldimethylether, Tetraethylenglykoldimethylether oder einen hochsiedenden aliphatischen oder aromatischen Kohlenwasserstoff oder beliebige Mischungen dieser Lösungsmittel verwenden. Besonders bevorzugt ist Tetramethylensulfon.

Bei der Auswahl des Lösungsmittels ist darauf zu achten, daß sein Siedepunkt beim angewendeten Druck höher ist als der Siedepunkt des herzustellenden Perfluoralkylaryl-sulfids der Formeln (I) oder (I').

Im allgemeinen setzt man pro mol Disulfid der Formeln (II) oder (II') 50 bis 1000 ml, vorzugsweise 100 bis 250 ml Lösungsmittel ein.

Die erfindungsgemäßen Verfahren werden unter einem Druck von 1 bis 500 mbar, vorzugsweise 10 bis 300 mbar, durchgeführt.

Die erfindungsgemäßen Verfahren werden bei einer Temperatur von 120 bis 250°C, vorzugsweise 180 bis 220°C, durchgeführt.

Optimale Reaktionstemperaturen und -drucke zur Herstellung eines bestimmten Produkts der Formeln (I) oder (I') kann man durch routinemäßige Vorversuche leicht ermitteln.

Man kann die erfindungsgemäßen Verfahren diskontinuierlich durchführen, z.B. indem man die gesamte Menge der Reaktanden und des Lösungsmittels vorlegt, den Druck vermindert, anschließend erwärmt, z.B. auf 120 bis 150°C und dann langsam bis zur Vervollständigung der Reaktion weiter aufheizt, wobei man gegebenenfalls den Druck weiter erniedrigen kann.

Die erfindungsgemäßen Verfahren kann man auch kontinuierlich durchführen, was bei größeren Ansätzen bevorzugt wird. Hierbei kann man z.B. eine kleine Menge der Edukte in einer für eine längere Zeit ausreichende Menge Lösungsmittel vorlegen, dann den Druck erniedrigen und die Reaktionstemperatur einstellen und anschließend, wenn die erhaltene Verbindung der Formel (I) oder (I") abzudestillieren beginnt, kontinuierlich oder in kürzeren Abständen portionsweise (= semikontinuierlich) Disulfid der Formel (II) oder (II') und Salz der Formel (III) hinzudosieren. Die Dosierung des Disulfids und des Salzes kann z.B. aus einem Vorratsbehälter erfolgen, der beide Komponenten in gemischter Form enthält. Man kann auch jede Komponente fiir sich aus getrennten Vorratsbehältern zudosieren, wobei die Dosierung beider Komponenten simultan oder taktartig in kürzeren Abständen zeitlich hintereinander erfolgen kann. Man kann eine oder beide Komponenten gegebenenfalls gemeinsam mit dem verwendeten Lösungsmittel zudosieren. Bei längerer Fahrweise ist es vorteilhaft, den Reaktorinhalt weitgehend konstant zu halten, indem man z.B. Teile des Reaktorinhalts kontinuierlich oder semikontinuierlich austrägt und durch frisches Lösungsmittel ersetzt.

Das aus dem Reaktionsgefäß abdestillierende Produkt der Formel (I) oder (I'') kann gegebenenfalls weiter aufgearbeitet werden unter Anwendung allgemein üblicher Methoden. Zur Abtrennung von gegebenenfalls vorhandenen Nebenprodukten und eventuell mitdestilliertem Lösungsmittel kann man z.B. fraktioniert redestillieren. Wasserlösliche Lösungsmittel kann man auch mit Wasser extrahieren. Das im Sumpf anfallende Alkalisalz eines Arylmercaptans kann durch Oxidation erneut in ein Disulfid der Formel (II) oder (II') überführt und dieses dann erneut in die Reaktion eingesetzt werden.

Die erfindungsgemäßen Verfahren haben die Vorteile, daß die Ausbeuten und die Reaktionskontrolle gegenüber den Verfahren des Standes der Technik spürbar verbessert und das Verfahren auch zur Einführung längerkettiger Perfluoralkylreste verwendet werden kann. Weiterhin erlaubt das erfindungsgemäße Verfahren eine kontinuierliche Fahrweise. Durch die erfindungsgemäße Herstellung von Verbindungen der Formel (I') werden diese in deutlich besseren Ausbeuten erhalten als bei der Umsetzung von Ausgangsverbindungen, die bereits Hydroxy-, Mercapto- oder Amino-Substituenten enthalten. Diese Ergebnisse sind außerordentlich überraschend.

Von den erfindungsgemäß herstellbaren Perfluoralkyl-aryl-sulfiden der Formel (I) sind 2-Nitrophenyl-pentafluorethyl-sulfid und 2-Aminophenyl-pentafluorethyl-sulfid neu. Mit ihnen werden weitere Verbindungen zur Verfügung gestellt, die die Möglichkeiten erweitern, ausgehend von Perfluoralkyl-aryl-sulfiden, Agrochemikalien, Pharmazeutika, Farbstoffe und Polymere analog den eingangs zitierten Literaturstellen herzustellen.

### Beispiele

### Beispiel 1

### 2 -Nitrophenyl-trifluonnethyl-sulfid

In einem 250 ml-Dreihalskolben ausgerüstet mit mechanischem Rührer, Vakuumdestillationsaufsatz und einem Feststoffdosiertrichter wurden 70 ml trockenes Tetramethylensulfon vorgelegt. Bei 40°C wurden dann unter Rühren 7 Gew.-% einer festen Mischung (vor dem Einfüllen in den Feststoffdosiertrichter gut vermischt) aus 120 g Bis-(2-nitrophenyl)-disulfid und 119 g wasserfreiem Kaliumtrifluoracetat zugesetzt und Vakuum angelegt. Bei einem Druck von 200 bis 230 mbar wurde auf 185 bis 190°C (Badtemperatur) erhitzt. Unter Aufrechterhaltung dieser Bedingungen wurde die feste Reaktandenmischung gleichmäßig im Verlauf von 40 min. zudosiert. Währenddessen wurden die flüchtigen Produkte in einer trockeneisgekühlten Vorlage mit nachgeschalteter Kühlfalle aufgefangen. Nach Beendigung der Reaktion wurden noch durch Absenken des Druckes auf 10 mbar bei 190°C (Badtemperatur) Reste ausdestilliert. Der Inhalt von Vorlage und Kühlfalle wurde auf 250 ml Wasser gegossen. Das Abtrennen der organischen Phase ergab 84 g (96 % d.Th.) gaschromatographisch reines 2-Nitrophenyl-trifluormethyl-sulfid (Siedepunkt: 105°C/12 mbar). Die ¹H-NMR-, ¹⁹F-NMR- und MS-Spektren bestätigten die Struktur.

### Beispiel 2

### 2-Aminophenyl-trifluormethyl-sulfid

Zu einer gerührten Lösung von 5,05 g 2-Nitrophenyl-trifluormethyl-sulfid erhalten nach Beispiel 1 in 30 ml Ethanol wurde tropfenweise eine Lösung von 25,53 g Zinn(II)-chlorid-dihydrat in 40 ml konz. Salzsäure gegeben. Nach Beendigung der Zugabe wurde auf 100°C erwärmt und 1,5 Stunden bei dieser Temperatur nachgerührt. Nach dem Abkühlen wurde Ethanol im Vakuum abgedampft und der Rückstand auf eine Mischung von 150 g Eis und 150 ml 40 gew.-%ige wäßrige Natronlauge gegossen. Das Produkt wurde mit Ether extrahiert. Die vereinigten Extrakte über Magnesiumsulfat getrocknet und im Vakuum eingeengt. Die Vakuumdestillation des Rückstandes lieferte 3,31 g (75,7 % d.Th.) 2-Aminophenyl-trifluormethyl-sulfid (Siedepunkt: 102-104°C/50 mbar, Schmelzpunkt: 30-31°C).
Die ¹H-NMR-, ¹⁹F-NMR- und MS-Spektren bestätigten die Struktur.
Insbesondere bei größeren Ansätzen oder kontinuierlicher Fahrweise kann die Reduktion auch katalytisch mit Wasserstoff durchgeführt werden.

### Beispiel 3

### 2-Methoxyphenyl-trifluormethyl-sulfid

Eine Mischung aus 6,04 g Bis-(2-methoxyphenyl)-disulfid, 6,60 g wasserfreiem Kaliumtrifluoracetat und 9 ml trockenem Tetramethylensulfon wurden in einer Vakuumdestillationsapparatur bei einem Druck von 65 mbar gerührt. Es wurde in einem Ölbad langsam bis max. 220°C erwärmt, währenddessen flüchtige Produkte in einer trockeneisgekühlten Vorlage aufgefangen wurden. Die Redestillation des Rohproduktes ergab 3,20 g (70 % d.Th.) analytisch reines 2-Methoxyphenyl-trifluormethyl-sulfid (Siedepunkt: 80°C/20 mbar).
Die ¹⁹F-NMR-Daten stimmten mit den J. Org. Chem. 59, 4047 (1985) publizierten überein. Darüber hinaus bestätigten die ¹H-NMR- und MS-Spektren die Struktur.

### Beispiel 4

### 2-Hydroxyphenyl-trifluormethyl-sulfid

Eine Mischung aus 4,69 g 2-Methoxyphenyl-trifluormethyl-sulfid erhalten nach Beispiel 3, 20 ml 48 gew.-%iger wäßriger Bromwasserstoffsäure und 20 ml 96 gew.-%iger Essigsäure wurden 24 Stunden unter Rückfluß gekocht. Danach wurde die Vollständigkeit der Reaktion gaschromatographisch überprüft. Nach dem Abkühlen der Reaktionsmischung wurde auf Wasser gegossen. Die organische Phase wurde abgetrennt und die wäßrige Phase mit Dichlormethan extrahiert. Der Extrakt wurde mit der organischen Phase vereinigt. Nach der Trocknung über Magnesiumsulfat, Einengen und Vakuumdestillation wurden 2,01 g (46 % d.Th.) 2-Hydroxyphenyl-trifluormethyl-sulfid (Siedepunkt: 65-66°C/26 mbar, Schmelzpunkt: 35-37°C) erhalten.
Die ¹H-NMR-, ¹⁹F-NMR- und MS-Spektren bestätigten die Struktur.

### Beispiel 5

### 2-Nitrophenyl-pentafluorethyl-sulfid

Analog Beispiel 3 wurde eine Mischung aus 12,24 g Bis-(2-nitrophenyl)-disulfid, 14,56 g wasserfreiem Kaliumpentafluorpropionat und 10 ml trockenem Tetramethylensulfon auf 190°C erhitzt, wobei das Vakuum während der Temperatursteigerung von 120 mbar bei 140°C auf zunächst 50 mbar bei 190°C und anschließend zur Vervollständigung der Destillation bei dieser Temperatur auf 10 mbar verbessert wurde. Die Redestillation des Rohproduktes ergab 8,13 g (75 % d.Th.) des bisher nicht bekannten 2-Nitrophenyl-pentafluorethyl-sulfids (Siedepunkt: 105 bis 107°C/14 mbar). Analytisch reines Produkt kann durch Waschen mit Wasser, anschließendem Trocknen über Magnesiumsulfat und erneuter Destillation erhalten werden.
- ¹H-NMR:: 400 MHz (CDCl₃): δ [ppm], J [Hz] 7,65 (td, 1H, H^{4od.5}, ³J_{HH} 7,6, ⁴J_{HH} = 2,0); 7,68 (td, 1H, H^{4 od. 5}, ³J_{HH} = 7,6, ⁴J_{HH} = 2,0); 7,90 (dd, 1H, H⁶, ³J_{HH} = 7,6, ⁴J_{HH} = 2.0); 8,00 (dd, 1H, H³, ³J_{HH} =7,6, ⁴J_{HH} = 2,0)
- ¹⁹F-NMR:: 376,3 MHz (CDCl₃): δ (CCl₃F) [ppm], J [Hz] -83,06 (t, 3F, CF₃, ³J_{FF} = 3,5); -91,12 (q, 2F, CF₂, ³J_{FF} = 3,5)
- MS:: [m/z] 273 M⁺; 154 (M-CF₂CF₃)⁺; 108 C₆H₄S⁺; 96 C₅H₄S⁺; 69 CF₃+

| C/H-Analyse [%]: | C | H | N |
|---|---|---|---|
| ber. für C₈H₄O₂NF₅S: | 35,17 | 1,48 | 5,13 |
| gef.: | 35,4 | 1,5 | 5,3 |

### Beispiel 6

### 2-Aminophenyl-pentafluormethyl-sulfid

Analog Beispiel 2 wurden aus 6,26 g 2-Nitrophenyl-pentafluorethyl-sulfid (erhalten gemäß Beispiel 5 und vorgelegt in 30 ml Ethanol) und 25,85 g Zinn(II)-chlorid-dihydrat in 40 ml konz. Salzsäure nach der Destillation 4,5 g (81 % d.Th.) des bisher nicht bekannten 2-Aminophenyl-pentafluorethyl-sulfids (Siedepunkt: 77°C/12 mbar) erhalten.
- ¹H-NMR:: 400 MHz (CDCl₃): δ [ppm], J [Hz] 4,38 (ws, 2H, NH₂), 6,70 (m, 2H, H³, H^{4 od.} 5); 7,24 (td, 1H, H^{4 od. 5}, ³J_{HH} = 7,6, ⁴J_{HH} = 1,5); 7,43 (dd, 1H, H⁶, ³J_{HH} = 7,6, ⁴J_{HH} = 1,5).
- ¹⁹F-NMR:: 376,3 MHz (CDCl₃): 8 (CCl₃F) [ppm], J [Hz] -83,24 (t, 3F, CF₃, ³J_{FF} = 3,5); -92,18 (q, 2F, CF₂, ³J_{FF} = 3,5).
- MS:: [m/z] 243 M⁺; 124 (M-CF₂CF₃)⁺; 80 C₅H₆N⁺;

| C/H-Analyse [%]: | C | H | N |
|---|---|---|---|
| ber. für C₈H₆NF₅S: | 39,51 | 2,49 | 5,76 |
| gef.: | 39,70 | 2,60 | 5,90 |

### Beispiel 7

### 4-Trifluormethylphenyl-trifluormethyl-sulfid

Analog zu Beispiel 3 wurde eine Mischung aus 12,10 g Bis-(4-trifluorphenyl)-disulfid, 10,39 g wasserfreiem Kaliumtrifluoracetat und 10 ml trockenem Tetramethylensulfon unter einem gleichzeitig von 120 mbar auf 50 mbar verbessertem Vakuum bis 195°C erhitzt Es wurden 12,61 g Rohdestillat erhalten, die anschließend auf Wasser gegossen wurden. Die organische Phase wurde abgetrennt, über Magnesiumsulfat getrocknet und redestilliert. Es wurden 5,24 g (63 % d.Th.) 4-Trifluormethylphenyl-trifluormethyl-sulfid mit einem Siedepunkt von 99-100°C/195 mbar und einem Schmelzpunkt von 30-31°C erhalten. Die ¹H-NMR-, ¹⁹F-NMR- und MS-Spektren bestätigten die Struktur.

### Beispiel 8

### 2-Chlor-5-nitrophenyl-trifluormethyl -sulfid

Analog Beispiel 1 wurden 45,43 g Bis-(2-Chlor-5-nitrophenyl)-disulfid mit 36,64 g wasserfreiem Kaliumtrifluoracetat und 25 ml trockenem Tetramethylensulfon umgesetzt und das Produkt aufgearbeitet. Es wurden 17,23 g einer Mischung aus 7 % 2-Fluor-5-nitrophenyl-trifluormethyl-disulfid und 93 % 2-Chlor-5-nitrophenyl-trifluormethyl-sulfid (GC-Analyse) erhalten. Die Destillation des Rohproduktes ergab reines 2-Chlor-5-nitrophenyl-trifluormethyl-sulfid (Siedepunkt: 126-129°C/16 mbar). Die ¹H-NMR-, ¹⁹F-NMR- und MS-Spektren bestätigten die Struktur.

### Beispiel 9

### 5-Amino-2-chlorphenyl-trifluormethyl-sulfid

Analog Beispiel 2 wurden aus 7,76 g 2-Chlor-5-nitrophenyl-trifluormethyl-sulfid (erhalten gemäß Beispiel 8) in 40 ml Ethanol und 33,98 g Zinn(II)-chlorid-dihydrat in 50 ml konz. Salzsäure nach Abdampfen des Diethylethers 5,75 g (84 % d.Th.) gaschromatographisch reines 5-Ammo-2-chlorphenyl-trifluormethyl-sulfid (Siedepunkt: 131-133°C/16 mbar) erhalten. Die ¹H-NMR-, ¹⁹F-NMR- und MS-Spektren bestätigten die Struktur.

### Beispiel 10 (Zum Vergleich)

Analog Tetrahedron Lett. 37, 9057 (1996) wurde bei Normaldruck eine Mischung aus 9,15 g Bis-(2-nitrophenyl)-disulfid, 9,15 g wasserfreiem Kaliumtrifluoracetat und 15 ml trockenem Tetramethylensulfon in einer Destillationsapparatur unter Rühren auf 230°C erhitzt, wobei flüchtige Produkte in einer trockeneisgekühlten (-78°C) Vorlage aufgefangen wurden. Das Rohprodukt wurde auf Wasser gegossen. Nach Phasentrennung wurden 1,63 g (24,6 % d.Th.) rohes 2-Nitrophenyl-trifluormethyl-sulfid erhalten.

## Patentansprüche

1. Verfahren zur Herstellung von Perfluoralkyl-aryl-sulfiden der Formel (I)
Ar-S-R^{f} (I),
in der
Ar für unsubstituiertes, einfach oder mehrfach substituiertes Phenyl, Naphthyl, Pyridyl oder Pyrimidyl steht, wobei die Substituenten unabhängig voneinander ausgewählt sind aus der Gruppe:
Halogen, Nitro, Cyano, C₁-C₆-Alkyl, durch Fluor und/oder Chlor substituiertes C₁-C₆-Alkyl, C₁-C₆-Alkoxy, Fluor und/oder Chlor substituiertes C₁-C₆-Alkoxy und wobei die Molekularmasse dieses Radikals, gegebenenfalls vorhandene Fluoratome nicht mitgerechnet, kleiner als 200 g/mol ist, und
R^{f} fiir ein Perfluoralkylradikal mit 1 bis 7 C-Atomen steht,
durch Umsetzung von Disulfiden der Formel (II)
Ar-S-S-Ar (II),
in der Ar die bei Formel (I) angegebene Bedeutung hat,
mit Alkalisalzen aliphatischer Perfluorcarbonsäuren der Formel (III)
R^{f}-COO⁽⁻⁾M⁽⁺⁾ (II),
in der
R^{f} für ein Perfluoralkylradikal mit 1 bis 7 C-Atomen steht und
M für ein Alkalimetall steht,
in Gegenwart von aprotischem Lösungsmittel, **dadurch gekennzeichnet, dass** man
- als aprotisches Lösungsmittel solche einsetzt, die einen höheren Siedepunkt als das jeweilige Produkt der Formel (I) haben,
- bei einem Druck von 1 bis 500 mbar und einer Temperatur von 120 bis 250°C arbeitet und
- das gebildete Produkt der Formel (I) in dem Maße abdestilliert, wie es sich bildet.

2. Verfahren zur Herstellung von Perfluoralkyl-aryl-sulfiden der Formel (I')
Ar'-S-R^{f} (I'),
in der
Ar' für durch Hydroxy, Mercapto oder Amino substituiertes Phenyl, Naphthyl, Pyridyl oder Pyrimidyl steht, wobei die Molekularmasse dieses Radikals kleiner als 200 g/mol ist, und
R^{f} für ein Perfluoralkylradikal mit 1 bis 7 C-Atomen steht,
durch Umsetzung mit Disulfiden der Formel (II')
Ar"-S-S-Ar" (II'),
in der
Ar" für durch Methoxy, Methylthio oder Nitro substituiertes Phenyl, Naphthyl, Pyridyl oder Pyrimidyl steht, wobei die Molekularmasse dieses Radikals kleiner als 200 g/mol ist,
mit Alkalisalzen aliphatischer Perfluorcarbonsäuren der Formel (III)
R^{f}-COO⁽⁻⁾M⁽⁺⁾ (III),
in der
R^{f} für ein Perfluoralkylradikal mit 1 bis 7 C-Atomen steht und
M für ein Alkalimetall steht,
in Gegenwart von aprotischem Lösungsmittel, **dadurch gekennzeichnet, dass** man
- als aprotisches Lösungsmittel solche einsetzt, die einen höheren Siedepunkt als das jeweilige Produkt der Formel (I) haben,
- bei einem Druck von 1 bis 500 mbar und einer Temperatur im Bereich 120-250°C arbeitet und
- das gebildete Produkt der Formel (I")
Ar"-S-R^{f} (Iʺ)
in der
Ar" die bei Formel (II') und
R^{f} für ein Perfluoralkylradikal mit 1 bis 7 C-Atomen steht,
in dem Maße abdestilliert, wie es sich bildet, und anschließend durch eine an sich bekannte Spaltung der Methoxy- oder Methylthiogruppe bzw. durch eine an sich bekannte Reduktion der Nitrogruppe Perfluoralkyl-aryl-sulfide der Formel (I') erhält.

3. Verfahren nach Ansprüchen 1 bis 2, **dadurch gekennzeichnet, dass** man pro mol Disulfid der Formel (II) oder (II') 1,0 bis 5 mol Alkalisalz einer Perfluorcarbonsäure der Formel (III) einsetzt.

4. Verfahren nach Ansprüchen 1 bis 3, **dadurch gekennzeichnet, dass** man als Lösungsmittel solche mit einem Siedepunkt bei Normaldruck von über 210°C einsetzt.

5. Verfahren nach Ansprüchen 1 bis 4, **dadurch gekennzeichnet, dass** man als Lösungsmittel Tetramethylensulfon, Triethylenglykoldimethylether, Tetraethylenglykoldimethylether oder einen aliphatischen oder aromatischen Kohlenwasserstoff oder beliebige Mischungen dieser Lösungsmittel verwendet, wobei der Siedepunkt des Lösungsmittels über dem Siedepunkt des Produktes liegt.

## Claims

1. Process for preparing perfluoroalkyl aryl sulphides of the formula (I)
Ar-S-R^{f} (I),
in which
Ar represents unsubstituted, mono- or polysub-stituted phenyl, naphthyl, pyridyl or pyrimidyl, where the substituents independently of one another are selected from the group consisting of:
halogen, nitro, cyano, C₁-C₆-alkyl, fluorine- and/or chlorine-substituted C₁-C₆-alkyl, C₁-C₆-alkoxy, fluorine- and/or chlorine-substituted C₁-C₆-alkoxy, where the molar mass of this radical, excluding any fluorine atoms that may be present, is less than 200 g/mol, and
R^{f} represents a perfluoroalkyl radical having 1 to 7 C atoms,
by reacting disulphides of the formula (II)
Ar-S-S-Ar (II),
in which Ar is as defined under formula (I),
with alkali metal salts of aliphatic perfluorocarboxylic acids of the formula (III)
R^{f}-COO⁽⁻⁾M⁽⁺⁾ (III),
in which
R^{f} represents a perfluoroalkyl radical having 1 to 7 C atoms and
M represents an alkali metal,
in the presence of an aprotic solvent, **characterized in that**
- the aprotic solvent used is a solvent whose boiling point is higher than that of the product of the formula (I) in question,
- the operation is carried out under a pressure of from 1 to 500 mbar and at a temperature from 120 to 250°C and
- the product of the formula (I) formed is distilled off at the rate at which it is formed.

2. Process for preparing perfluoroalkyl aryl sulphides of the formula (I')
Ar'-S-R^{f} (I'),
in which
Ar' represents in each case hydroxyl-, mercapto- or amino-substituted phenyl, naphthyl, pyridyl or pyrimidyl, where the molar mass of this radical is less than 200 g/mol, and
R^{f} represents a perfluoroalkyl radical having 1 to 7 C atoms,
by reaction with disulphides of the formula (II')
Ar"-S-S-Ar" (II'),
in which
Ar" represents in each case methoxy-, methylthio- or nitro-substituted phenyl, naphthyl, pyridyl or pyrimidyl, where the molar mass of this radical is less than 200 g/mol,
with alkali metal salts of aliphatic perfluoro-carboxylic acids of the formula (III)
R^{f}-COO(⁻)M(⁺) (III),
in which
R^{f} represents a perfluoroalkyl radical having 1 to 7 C atoms and
M represents an alkali metal,
in the presence of an aprotic solvent, **characterized in that**
- the aprotic solvent used has a boiling point higher than that of the product of the formula (I) in question,
- the operation is carried out at a pressure of from 1 to 500 mbar and at a temperature in the range 120-250°C
- the product of the formula (I") formed
Ar"-S-R^{f} (Iʺ)
in which
Ar" is as defined under formula (II') and
R^{f} represents a perfluoroalkyl radical having 1 to 7 C atoms,
is distilled off at the rate at which it is formed, and perfluoroalkyl aryl sulphides of the formula (I') are subsequently obtained by a cleavage, which is known per se, of the methoxy or methylthio group, or by a reduction, which is known per se, of the nitro group.

3. Process according to Claims 1 and 2, **characterized in that** from 1.0 to 5 mol of alkali metal salt of a perfluorocarboxylic acid of the formula (III) are employed per mole of disulphide of the formula (II) or (II').

4. Process according to Claims 1 to 3, **characterized in that** the solvent used has a boiling point at atmospheric pressure of above 210°C.

5. Process according to Claims 1 to 4, **characterized in that** the solvent used is tetramethylene sulphone, triethylene glycol dimethyl ether, tetraethylene glycol dimethyl ether or an aliphatic or aromatic hydrocarbon or any mixture of these solvents, the boiling point of the solvent being above the boiling point of the product.

## Revendications

1. Procédé pour la préparation de sulfures de perfluoralkyle et d'aryle de formule (I)
Ar-S-Rf (I),
dans laquelle
Ar représente un groupe phényle, naphtyle, pyridyle ou pyrimidyle non substitué ou portant un ou plusieurs substituants lesquels sont choisis, indépendamment les uns des autres, dans le groupe suivant :
halogéno, nitro, cyano, alkyle en C₁-C₆, alkyle en C₁-C₆ portant lui-même des substituants fluoro et/ou chloro, alcoxy en C₁-C₆, alcoxy en C₁-C₆ portant lui-même des substituants fluoro et/ou chloro, la masse moléculaire de ce radical, non comptés les atomes de fluor éventuellement présents, étant inférieure à 200 g/mol, et
R^{f} représente un radical perfluoralkyle en C₁-C₇,
par réaction de disulfures de formule (II)
Ar-S-S-Ar (II),
dans laquelle Ar a les significations indiquées en référence à la formule (I), avec des sels alcalins d'acides perfluorocarboxyliques aliphatiques de formule (III)
R^{f}-COO(-)M(+) (III),
dans laquelle
R^{f} représente un radical perfluoralkyle en C₁-C₇ et
M représente un métal alcalin,
en présence d'un solvant aprotonique, **caractérisé en ce que**
- on utilise un solvant aprotonique ayant un point d'ébullition plus élevé que le produit de formule (I),
- on opère à une pression de 1 à 500 mbar et à une température de 120 à 250°C et
- on distille le produit de formule (I) formé au fur et à mesure de sa formation.

2. Procédé pour la préparation de sulfures de perfluoralkyle et d'aryle de formule (I')
Ar'-S-Rf (I'),
dans laquelle
Ar' représente un groupe phényle, naphtyle, pyridyle ou pyrimidyle portant des substituants hydroxy, mercapto ou amino, la masse moléculaire de ce radical étant inférieure à 200 g/mol, et
R^{f} représente un radical perfluoralkyle en C₁-C₇,
par réaction de disulfures de formule (II')
Ar"-S-S-Ar" (II'),
dans laquelle
Ar" représente un groupe phényle, naphtyle, pyridyle ou pyrimidyle portant des substituants méthoxy, méthylthio ou nitro, la masse moléculaire de ce radical étant inférieure à 200 g/mol,
avec des sels alcalins d'acides perfluorocarboxyliques aliphatiques de formule (III)
Rf-COO⁽⁻⁾M⁽⁺⁾ (III),
dans laquelle
R^{f} représente un radical perfluoralkyle en C₁-C₇ et
M représente un métal alcalin,
en présence d'un solvant aprotonique **caractérisé en ce que**
- on utilise un solvant aprotonique dont le point d'ébullition est plus élevé que celui du produit de formule (I),
- on opère à une pression de 1 à 500 mbar et à une température dans l'intervalle de 120 à 250°C,
- on distille le produit formé, de formule (I")
Ar"-S-R^{f} (Iʺ)
dans laquelle
Ar" a les significations indiquées en référence à la formule (II") et
R^{f} représente un radical perfluoralkyle en C₁-C₇,
au fur et à mesure de sa formation, après quoi on obtient les sulfures de perfluoralkyle et d'aryle de formule (I') par une scission, connue en soi, du groupe méthoxy ou méthylthio et par une réduction, connue en soi du groupe nitro.

3. Procédé selon les revendications 1 à 2, **caractérisé en ce que** pour 1 mol de disulfure de formule (II) ou (II'), on utilise 1,0 à 5 mol du sel alcalin d'acide perfluorocarboxylique de formule (III).

4. Procédé selon les revendications 1 à 3, **caractérisé en ce que** l'on utilise un solvant ayant un point d'ébullition à pression normale supérieur à 210°C.

5. Procédé selon les revendications 1 à 4, **caractérisé en ce que** le solvant utilisé est la tétraméthylènesulfone, l'éther diméthylique du triéthylèneglycol, l'éther diméthylique du tétraéthylèneglycol ou un hydrocarbure aliphatique ou aromatique ou un mélange quelconque de ces solvants, dont le point d'ébullition est supérieur au point d'ébullition du produit.
